# EUROPÄISCHE PATENTSCHRIFT

(11) **EP 0 439 028 B1**
(45) Veröffentlichungstag und Bekanntmachung des Hinweises auf die Patenterteilung: **25.05.1994**
(21) Anmeldenummer: 91100297.0
(22) Anmeldetag: 11.01.1991
(51) Int. Cl.: A61F 2/64, A61F 5/01

(54) **Schwenkverbindung zwischen zwei Teilen eines orthopädie-technischen Hilfsmittels**
Swivelling connection between two parts of an orthopaedic device
Liaison pivotante entre deux parties d'un dispositif orthopédique

(30) Priorität: 26.01.1990 NL 9000195; 19.02.1990 DE 4004988
(43) Veröffentlichungstag der Anmeldung: 31.07.1991
(73) Patentinhaber: Otto Bock Orthopädische Industrie Besitz- und Verwaltungs-Kommanditgesellschaft, 37115 Duderstadt (DE)
(72) Erfinder: van de Veen, Paul Gerard, Dr. Ir., NL-7514 EC Enschede (NL)
(74) Vertreter: Gramm, Werner, Prof., Dipl.-Ing.

(56) Entgegenhaltungen:
- EP-A- 0 010 177
- EP-A- 0 243 081
- DE-A- 1 491 233
- DE-A- 1 766 738
- DE-C- 811 254
- DE-C- 828 292
- FR-A- 1 035 104
- FR-A- 2 344 271
- GB-A- 285 737
- GB-A- 1 533 796
- US-A- 4 064 569

## Beschreibung

Die Erfindung betrifft eine Schwenkverbindung zwischen zwei Teilen eines orthopädie-technischen Hilfsmittels, z.B. einer Prothese oder Orthese, mit einer einrichtbaren, inhärenten Gelenk-Grundstabilität, bestehend aus einer ebenen kinematischen Gelenkkette mit zumindest vier Gelenkgliedern und einer sich aus translatorischen und rotatorischen Komponenten zusammensetzenden polyzentrischen Verschwenkcharakteristik, wobei die Verschwenkung zumindest eines Gelenkgliedes in zumindest einer Schwenkrichtung durch einen ersten Schwenkanschlag begrenzt ist. Die Gelenkglieder können einzeln oder paarweise ausgebildet sein.

Derartige Schwenkverbindungen (siehe z.B. EP-A-0 010 177) werden z.B. als prothesenkniegelenk eingesetzt. Ihre Beugecharakteristik ist durch eine sich aus translatorischen und rotatorischen Komponenten zusammensetzende polyzentrische Bewegung gekennzeichnet, die dem Abrollen zweier Kurven aufeinander entspricht, deren jeweiliger Berührungspunkt den Momentandrehpunkt der Bewegung darstellt. Jede dieser sogenannten Polkurven ist einem der mittels der polyzentrischen Schwenkverbindung miteinander verbundenen Teile der Prothese zugeordnet. Derartige polyzentrische Kniegelenke können je nach Auslegung ihrer Grundgeometrie - neben Vorteilen in der Sitzposition - vor allem während der Standphase des Gehens (gerechnet vom Fersenkontakt bis zum Abheben der Fußspitze) gegenüber monozentrischen, also einachsigen Kniegelenkausführungen besondere Vorteile bieten.

Ein monozentrisches Prothesenkniegelenk, das durch ausschließliche Rückverlagerung hinter die Belastungslinie beim Stehen statisch gesichert ist, wird unter Fersenlast zu Beginn der Standphase des Gehens aufgrund der veränderten Belastungsrichtung instabil und muß demzufolge vom Amputierten durch Einleiten eines Hüftstreckmomentes gegen Einknicken gesichert werden. Die Höhe dieses Momentes ist abhängig von der Größe der Rückverlagerung des Kniegelenkes. Die physische Anstrengung des Amputierten läßt sich demzufolge nur durch Reduzierung der Rückverlagerung mindern, die ihrerseits wegen des damit verbundenen Stabilitätsverlustes kompensatorische Maßnahmen wie z.B. die Integration zusätzlicher Standphasen-Sicherungselemente in Gestalt mechanischer Bremsen oder hydraulischer Dämpfer erfordert.

Demgegenüber kann ein polyzentrisches Prothesenkniegelenk bei entsprechend hoher Lage des Momentandrehpunktes der Strecklage nicht nur beim Stehen sondern auch unter Fersenlast zu Beginn der Standphase des Gehens eine so ausgeprägte inhärente Stabilität aufweisen, daß es in gestreckter Stellung auch ohne Hüftstreckmoment sicher ist. Ein weiterer Vorteil läßt sich bei entsprechender Auslegung am Ende der Standphase erreichen, an dem das natürliche Gangbild zur harmonischen Überleitung in die anschließende Schwungphase die Einleitung einer Kniebeugung unter Vorfußlast vorsieht. Dies ließe sich mit einer Prothese mit rückverlagertem, monozentrischem Kniegelenk nicht nachvollziehen, da ein Durchschnittsamputierter das dazu erforderliche Hüftbeugemoment auf Dauer kaum aufbringen kann. Demgegenüber erfordert die Beugung einer mit einem polyzentrischen Kniegelenk der vorgenannten Auslegung ausgestatteten Prothese unter Vorflußlast am Ende der Stanphase ein weit geringeres Hüftbeugemoment, das vom Durchschnittsamputierten ohne Ermüdung aufgebracht werden kann. Diese Vorteile ließen sich jedoch nur dann realisieren, wenn für den Momentandrehpunkt der Strecklage die jeweils optimale Position einstellbar wäre.

Den Vorteilen eines polyzentrischen Prothesenkniegelenkes mit den vorerwähnten funktionellen Merkmalen steht jedoch in der Regel ein Nachteil gegenüber: Es verliert seine in der Strecklage ausgeprägte inhärente Stabilität bereits bei geringen Beugewinkeln. Deshalb ist es bisher nicht möglich, eine Beinprothese so zu gestalten, daß sie dem Amputierten über die nur mit polyzentrischen Prothesenkniegelenken erreichbaren vorerwähnten Vorteile hinaus die zusätzliche Möglichkeit bietet, in Annäherung an das natürliche Gangbild zu Beginn der Stanphase des Gehens unter Fersenlast eine elastisch abgefederte und/oder gedämpfte Kniebeugung ohne Stabilitätsverlust einzuleiten, um einerseits eine Verbesserung von Gangbild und Ergonomie die Vertikalbewegung seines Körperschwerpunktes im gesamten Verlauf der Standphase ausgewogener und harmonischer zu gestalten.

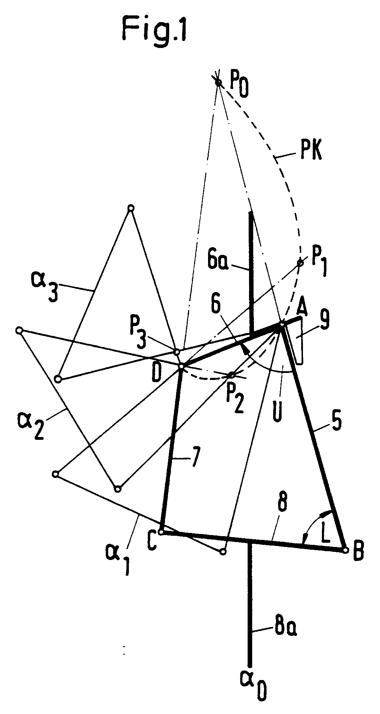

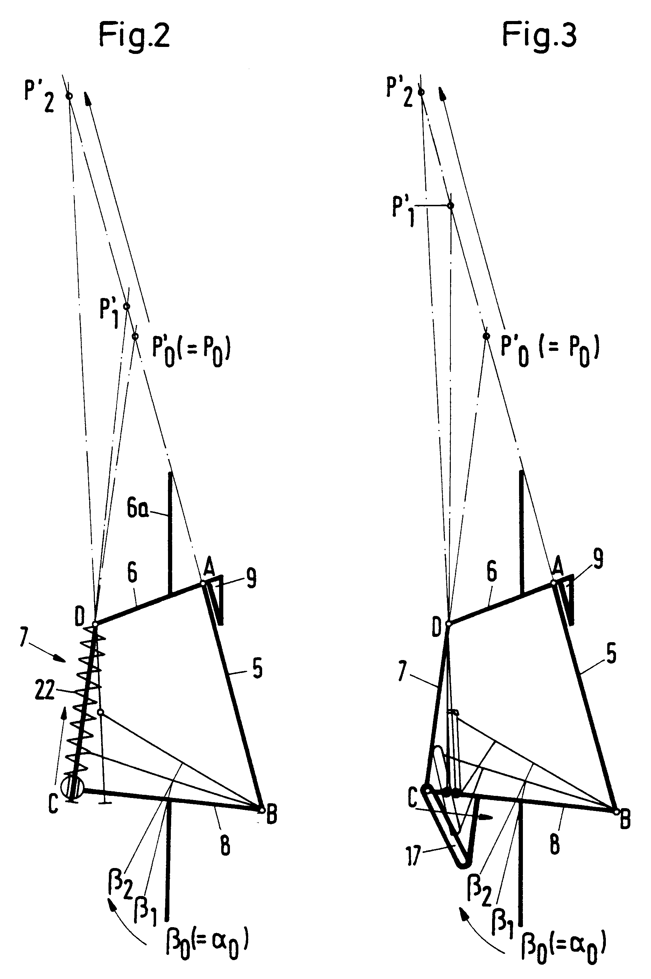

Die DE-A-1 766 738 offenbart ein polyzentrisches ProthesenKniegelenk, dessen getriebetechnisches Konzept als sogenannte offene ebene kinematische Gelenkkette mit zwangsläufiger Punktkurvenführung zu bezeichnen ist. Die die kinematische Charakteristik bestimmte Gelenkmechanik setzt sich bei den in den Figuren 1 und 2 der Vorveröffentlichung gezeigten Ausführungsbeispielen zusammen aus dem Gelenkoberteil 1, das mit dem oberen Ende des hinteren Gelenkgliedes 16 durch das Drehgelenk 11 schwenkbar verbunden ist, und dessen Unterseite ein im hinteren Bereich annähernd kreisförmiges, nach vorne gerade auslaufenden Kurvenprofil mit Gleitblech 17 aufweist, sowie aus dem Gelenkunterteil 2, das mit dem unteren Ende des hinteren Gelenkgliedes 16 durch das Drehgelenk 12 schwenkbar verbunden ist, und das sich mit den Stützflächen 3,4 am Kurvenprofil an der Unterseite des Gelenkoberteils 1 abstützt. Zur Aufrechterhaltung des konstruktiv vorgesehenen Zwangslaufes (1 Freiheitsgrad) dürfen die Stützflächen 3,4 zu keinem Zeitpunkt der Nutzung des Prothesen-Kniegelenkes vom Kurvenprofil abheben. Dies wird in erster Linie dadurch erreicht, daß die Stützflächen 3,4 als Wippe gestaltet sind. Ein auf der Gelenkvorderseite zwischen den Befestigungspunkten 9 und 10 im Gelenkunter- bzw. -oberteil wirkendes elastisches Band 8 dient als sogenannter Vorbringer und ist so angelenkt, daß sich seine Streckwirkung in eine Beugewirkung umkehrt, sobald es bei gebeugtem Kniegelenk hinter dem Momentanzentrum der polyzentrischen Bewegung verläuft, das bereits bei relativ geringen Beugewinkeln annähernd mit dem Drehpunkt 11 übereinstimmt. Das elastische Band 8 stellt somit aus getriebetechnischer Sicht kein Gelenkglied der kinematischen Kette dar, da es keinerlei Einfluß auf deren kinematische Charakteristik ausübt.

Die FR-A-1 035 104 offenbart ein polyzentrisches Prothesen-Kniegelenk, dessen getriebetechnisches Konzept dem der DE-A-1 766 738 entspricht. Die die kinematische Charakteristik bestimmende Gelenkmechanik setzt sich hier zusammen aus dem Gelenkoberteil 1, das mit dem oberen Ende des hinteren Gelenkgliedes 4 durch das Drehgelenk 2 schwenkbar verbunden ist, und dessen vorderes unteres Ende ein Kurvenprofil aufweist, sowie aus dem Gelenkunterteil 7, das mit dem unteren Ende des hinteren Gelenkgliedes 4 durch das Drehgelenk 8 schwenkbar verbunden ist und sich mit der Stützrolle 11 am Kurvenprofil am vorderen unteren Ende des Gelenkoberteils abstützt. Zur Aufrechterhaltung des konstruktiv vorgesehenen Zwangslaufes (Freiheitsgrad = 1) ist dafür gesorgt, daß die Stützrolle 11 zu keinem Zeitpunkt der Nutzung des Prothesen-Kniegelenkes vom Kurvenprofil abhebt. Dies wird in erster Linie erreicht durch den elastischen Zuggurt 14. Dieser Gurt 14 stellt jedoch aus getriebetechnischer Sicht kein Gelenkglied der kinematischen Kette dar, da er keinen Einfluß auf ihre kinematische Charakteristik ausübt. Die aus Drehgelenk 3, Hebel 5 sowie dem an letzterem schwenkbar angelenkten Schubgelenk mit den Gliedern 9,10 bestehende Mechanik dient dazu, bei Einleitung einer Kniebeugung mit Winkeln von 20 bis 30 (160 bis 150) Grad aus der Strecklage bei Null (180) Grad, wobei sich das Kurvenprofil am vorderen unteren Ende des Gelenkoberteils 1 entlang der am Gelenkunterteil 7 ortsfest gelagerten Stützrolle 11 vom Stützpunkt a in Richtung Stützpunkt b bewegt, den federelastischen Puffer 15 zu komprimieren und dadurch die vorgenannte Kniebeugung elastisch abzufedern. Diese Zusatz-Mechanik stellt jedoch aus getriebetechnischer Sicht kein Gelenkglied der kinematischen Kette dar, da sie keinen Einfluß auf ihre kinematische Charakteristik ausübt.

Die EP-A-0 010 177 offenbart ein polyzentrisches ProthesenKniegelenk in Gestalt einer viergliedrigen ebenen kinematischen Gelenkkette mit Zwangslauf (Freiheitsgrad = 1). Diese kinematische Gelenkkette besteht aus einem Gelenkoberteil 1 und einem Gelenkunterteil 4, die jeweils ein vorderes und ein hinteres Drehgelenk 8 bis 11 aufweisen, von denen jeweils die beiden vorderen und die beiden hinteren durch gekrümmte Lenker 5,6 miteinander verbunden sind. Zwischen Gelenkunterteil 4 und hinterem Lenker 6 ist ein Vorbringersystem 19 angelenkt. Vorgesehen ist ferner ein auf den vorderen Lenker wirkender justierbarer Streckanschlag 27,28.

Der Erfindung liegt die Aufgabe zugrunde, die Funktion einer polyzentrischen Schwenkverbindung zu erweitern.

Diese Aufbabe wird gemäß der Erfindung dadurch gelöst, daß unter Einwirkung einer äußeren Kraft zumindest ein Gelenkglied derart längenveränderlich ausgebildet ist, daß sich die Verschwenkcharakteristik in Abhängigkeit der äußeren Kraft (F) ändert. Diese Längenveränderung kann z. B. dadurch erzielt werden, daß ein zwei Gelenkglieder miteinander verbindender Gelenkpunkt unter Belastung in Richtung des einen und/oder des anderen Gelenkgliedes verschiebbar ist.

Fallweise wird eine der genannten Längenänderung entgegenwirkende Widerstandseinrichtung vorgesehen, die z.B. eine mechanische Reibung erzeugende Einrichtung, ein Dämpfer o.dergl. sein kann.

Besondere Vorteile lassen sich erzielen, wenn die Längenveränderung des zumindest einen Gelenkgliedes federelastisch ausgebildet ist. Bei dieser Ausbildung erfolgt die Längenveränderung jeweils gegen die Wirkung einer Rückstellkraft. Hierfür können Federn, gummielastische oder pneumatische Einrichtungen o.dergl. vorgesehen werden. Grundsätzlich besteht auch die Möglichkeit, das längenveränderliche Gelenkglied als Blattfeder auszubilden.

In beispielsweiser Auslegung der erfindungsgemäßen Konstruktion ergibt sich durch die belastungsabhängige Verkürzung des Abstandes zweier benachbarter Gelenkpunkte eine belastungsabhängige beugeseitige Verschwenkung des Gelenkunterteils zum Gelenkoberteil bei gleichzeitiger Verlagerung des Momentandrehpunktes weg von der Schwenkverbindung auf einer zu ihrer Beugeseite geneigten Geraden, die mit der Längsachse desjenigen Gelenkgliedes fluchtet, dessen durch den Schwenkanschlag vorgegebene Grenzlage erhalten bleibt.

Diese belastungsabhängige Zusatzkinematik führt zu einer Erhöhung der einrichtbaren inhärenten Gelenk-Grundstabilität, wobei sich mit der erfindungsgemäßen Konstruktion durch einfache getriebetechnische Variationen die einrichtbare inhärente Gelenk-Grundstabilität auch in anderer Weise beeinflussen läßt: Die erfindungsgemäße Schwenkverbindung ist generell durch die Fähigkeit gekennzeichnet, unter Einwirkung einer äußeren Belastung eine begrenz- und kontrollierbare Zusatzbewegung in einer vorgegebenen Richtung zu ermöglichen und dabei gleichzeitig die einrichtbare inhärente Gelenk-Grundstabilität je nach konstruktiver Auslegung in gezielter Weise im Sinne einer ausschließlichen Verminderung, Gleichhaltung oder Steigerung oder im Sinne einer zusatzbewegungsabhängigen Kombination dieser Wirkungen qualitativ und quantitativ zu beeinflussen.

Soweit also in der nachfolgenden Beschreibung auf ein Kniegelenk Bezug genommen wird, handelt es sich lediglich um eine beispielsweise, wenn auch besonders vorteilhafte Anwendung der erfindungsgemäßen Schwenkverbindung.

Bei Verwendung als Kniegelenk bietet die erfindungsgemäße Schwenkverbindung erstmals die Möglichkeit einer Kniebeugung unter Fersenlast zu Beginn der Standphase des Gehens ohne jede Einschränkung der vorstehend in Verbindung mit den vorbekannten polyzentrischen Prothesenkniegelenken erläuterten Vorteile. Ein erfindungsgemäß ausgebildetes Prothesenkniegelenk ist somit den als "bouncing knees" bekanntgewordenen monozentrischen Ausführungen funktionell deutlich überlegen.

Für die Einstellung der Gelenkstabilität ist es vorteilhaft, wenn der erste Schwenkanschlag justierbar ist. Die größtmögliche Freizügigkeit für die Einstellung der Gelenkstabilität ist jedoch erst dann erreichbar, wenn zusätzliche Maßnahmen, z.B. eine manuelle Längenveränderung eines Gelenkgliedes vorgesehen werden.

Die Stabilitätsverbesserung der belasteten Schwenkverbindung ist insbesondere bei Verwendung als Kniegelenk dann besonders signifikant, wenn der erste Schwenkanschlag zwischen zwei starr ausgebildeten Gelenkgliedern angeordnet ist, wobei eines dieser beiden Gelenkglieder vorzugsweise durch das obere Verbindungsteil gebildet wird.

Bei einer Anwendung der erfindungsgemäßen Schwenkverbindung als Kniegelenk bilden das erste Teil des orthopädie-technischen Hilfsmittels einen Prothesenoberschenkel, das zweite Teil einen Prothesenunterschenkel und eine viergliedrige Gelenkkette das Kniegelenk, dessen die beiden mit dem Prothesenober- bzw. -unterschenkel fest verbundenen Gelenkglieder über ein vorderes, für die Vorgabe der Strecklage genutztes Gelenkglied (streckseitiges Koppelglied) sowie über ein hinteres Gelenkglied (beugeseitiges Koppelglied) über Gelenkpunkte miteinander verbunden sind. Erfindungsgemäß ist dann vorzugsweise das beugeseitige Koppelglied das unter Belastung federelastisch längenveränderliche Gelenkglied.

Zusätzlich ist es zweckmäßig, wenn der das beugeseitige Koppelglied mit dem unteren Gelenkglied verbindende Gelenkpunkt innerhalb des unteren Gelenkgliedes längsverschiebbar angeordnet und in der gewünschten Position fixierbar ist.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung ist die Erfindung schematisch erläutert und anhand eines speziellen Ausführungsbeispieles näher beschrieben. Es zeigen:
- Figur 1: ein in Strecklage dargestelltes polyzentrisches Gelenk gemäß dem Stand der Technik;
- Figur 2: in einer Darstellung gemäß Figur 1 eine polyzentrische Schwenkverbindung gemäß der Erfindung;
- Figur 3: eine abgewandelte Ausführungsform in einer Darstellung gemäß Figur 2;
- Figur 4: eine abgewandelte Ausführungsform in einer Darstellung gemäß Figur 3;
- Figur 5: in einer Darstellung gemäß Figur 2 in Gegenüberstellung die Wirkungen einer belastungsunabhängigen Grundkinematik und einer belastungsabhängigen Zusatzkinematik;
- Figur 6: die Ausführungsform gemäß Figur 5 als Prothesenkniegelenk;
- Figur 7: in perspektivischer Darstellung eine Beinprothese mit einem Prothesenkniegelenk gemäß Figur 6;
- Figur 8: in perspektivischer Darstellung und zum Teil in Explosionsdarstellung einen Ausschnitt des Kniegelenkes gemäß Figur 7;
- Figur 9: in vergrößertem Maßstab in Seitenansicht einen Ausschnitt der Figur 7 mit eingezeichnetem Verschwenkbereich und
- Figur 10: die Darstellung gemäß Figur 9 in Beugestellung.

Figur 1 zeigt schematisch eine sich in gestreckter Position befindliche Schwenkverbindung, bestehend aus einer ebenen kinematischen Gelenkkette mit vier Gelenkgliedern 5,6,7,8, die über vier Gelenkpunkte A,B,C,D miteinander verbunden sind. Dabei ist das obere Gelenkglied 6 mittels einer schematisch dargestellten Anschlußvorrichtung 6a mit dem oberen Teil eines orthopädie-technischen Hilfsmittels verbindbar. In gleicher Weise weist das untere Gelenkglied 8 eine Anschlußvorrichtung 8a für das untere Teil eines orthopädie-technischen Hilfsmittels auf. Bei dem in Figur 1 rechts liegenden Gelenkglied 5 handelt es sich um ein streckseitig angeordnetes Koppelglied, dem ein beugeseitig angeordnetes, durch das links dargestellte Gelenkglied 7 gebildetes Koppelglied gegenüberliegt. Die Strecklage dieser Schwenkbewegung wird durch einen Anschlag 9 definiert, der bei der dargestellten Ausführungsform das Maximum des zwischen dem oberen Gelenkglied 6 und dem streckseitigen Koppelglied 5 eingeschlossenen Winkels U begrenzt.

Es handelt sich um eine vorbekannte Schwenkverbindung, bei der die Abstände zwischen den benachbarten Gelenkpunkten jeweils unveränderbar sind. Die Beugecharakteristik ist ausgehend von der in dick ausgezogenen Linien dargestellten gestreckten Position (α0) in drei Beugestufen (α1, α2, α3) des Gelenkunterteils zum ruhenden Gelenkoberteil dargestellt. Diese Beugecharakteristik setzt sich zusammen aus einer translatorischen und einer rotatorischen Komponente und führt zu einer polyzentrischen Bewegung, die durch die gestrichelt eingezeichnete, dem ruhenden Gelenkoberteil zugeordnete Polkurve PK (Rastpolbahn) beschrieben wird, die alle den jeweiligen Beugestellungen zugehörigen Momentandrehpunkte P miteinander verbindet, von denen im dargestellten Beispiel der der Strecklage zugehörige Momentandrehpunkt P₀ am weitesten oberhalb des Gelenkes liegt.

Figur 2 zeigt eine Schwenkverbindung gemäß der Erfindung. Im Unterschied zur Ausführungsform gemäß Figur 1 ist hier das beugeseitige Koppelglied 7 unter Einwirkung einer äußeren, in der Zeichnung nicht näher dargestellten Druckkraft federelastisch längenveränderlich ausgebildet, indem sein unterer Gelenkpunkt C gegen die Wirkung einer Druckfeder 22 in Richtung seines oberen Gelenkpunktes D verschiebbar ist. Ausgehend von der belastungsfreien Ausgangsstellung (β0 = α0) sind zwei belastungsabhängige Verschiebepositionen des Gelenkpunktes C eingezeichnet, die zu einer Verschwenkung des unteren Gelenkgliedes 8 um den Winkel β1 bzw. β2 und zu einer Verschwenkung des beugeseitigen Koppelgliedes7 um seinen oberen Gelenkpunkt D führen. Im Vergleich zu der Darstellung gemäß Figur 1 handelt es sich hier um eine belastungsabhängige Zusatzkinematik, die in den Zeichnungen jeweils mit dem Winkel β gekennzeichnet ist, und die dadurch erkennbar ist, daß die durch den Anschlag 9 vorgegebene Winkelposition zwischen den starr ausgebildeten Gelenkgliedern 5,6 erhalten bleibt.

Die belastungsabhängige Zusatzkinematik bewirkt ihrerseits eine Beugebewegung des Unterteils der Schwenkverbindung gegenüber dem ruhenden Oberteil bei gleichzeitiger Verlagerung des Momentandrehpunktes P, der jeweils definiert ist durch den Schnittpunkt der Verlängerungslinien der beiden Koppelglieder 5,7. Dabei erfolgt die Verlagerung der Momentandrehpunkte P in Abhängigkeit von der Beugestellung β des Unterteils auf einer Geraden, die die Verlängerung der Längsachse desjenigen der Koppelglieder 5,7 darstellt, dessen durch den Anschlag 9 vorgegebene Grenzlage erhalten bleibt. Bei dem Ausführungsbeispiel gemäß Figur 2 liegen somit alle Momentandrehpunkte P′₀, P′₁ und P′₂ auf der strichpunktiert eingezeichneten Verlängerung des Koppelgliedes 5, wobei sich bei dem gewählten Beispiel die Momentandrehpunkte P′ mit zunehmendem Beugewinkel β vom Gelenk entfernen. Dieser Wirkung der erfindungsgemäßen Lösung kommt die größte Bedeutung zu.

Das Ausführungsbeispiel gemäß Figur 3 unterscheidet sich von dem gemäß Figur 2 lediglich darin, daß der Gelenkpunkt C nicht in Richtung auf den Gelenkpunkt D sondern in Richtung auf den Gelenkpunkt B gegen die Wirkung einer nicht dargestellten Feder o.dergl. unter Belastung verschiebbar ist. Die Verschiebung des Gelenkpunktes C erfolgt dabei innerhalb eines Längsschlitzes 17 derart, daß sich eine belastungsabhängige Verschwenkung des unteren Gelenkgliedes 8 sowie eine Verlagerung des Momentandrehpunktes P analog Figur 2 ergeben.

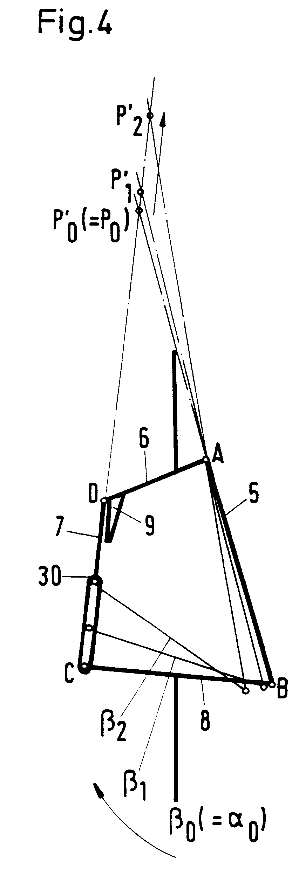

Figur 4 zeigt eine der Figur 2 vergleichbare Ausführungsform, wobei lediglich der Anschlag 9 nunmehr die Verschwenkung des beugeseitigen Koppelgliedes 7 gegenüber dem oberen Gelenkglied 6 begrenzt. Die Verlagerung der Momentandrehpunkte P′ in Abhängigkeit von der Beugestellung β des Unterteils der Schwenkverbindung gegenüber dem ruhenden Oberteil erfolgt somit auf der strichpunktiert eingezeichneten Verlängerung des beugeseitigen Koppelgliedes 7. Ferner ist ein Längenanschlag 30 angedeutet, der das Maximum der federelastischen Verkürzung des beugeseitigen Koppelgliedes 7 bzw. der Verschiebung des Gelenkpunktes C gegenüber dem Gelenkpunkt D begrenzt.

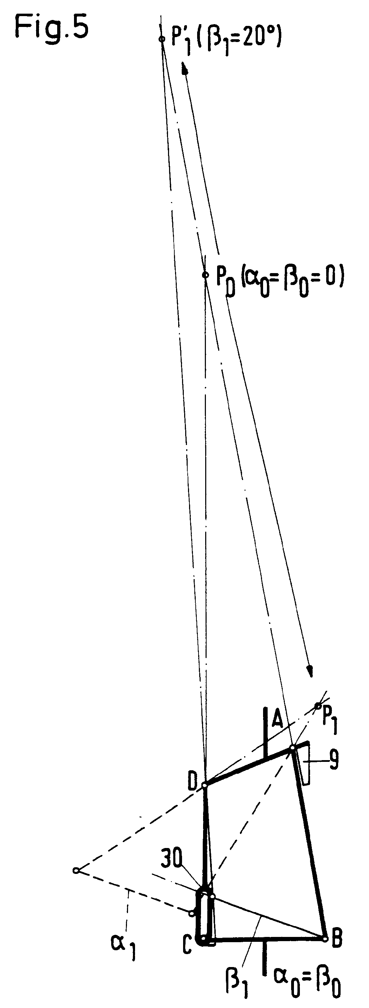

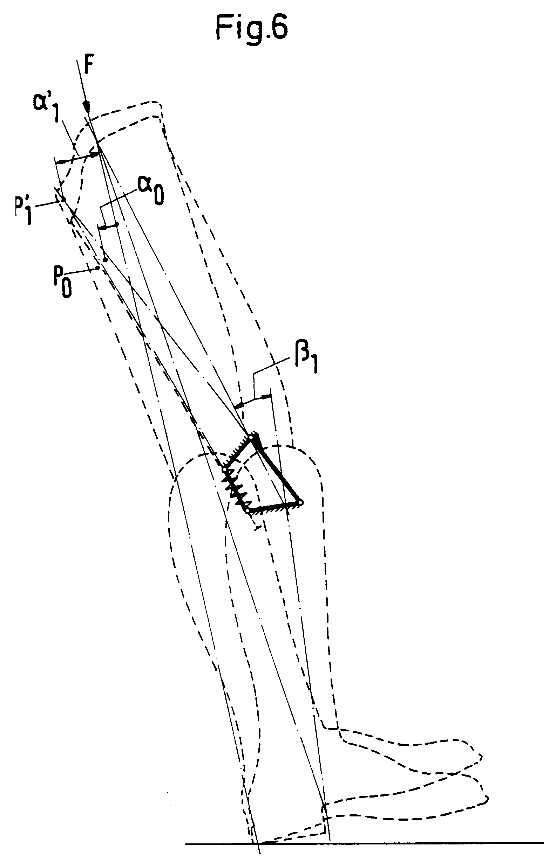

Figur 5 zeigt eine Schwenkverbindung gemäß Figur 2 und macht den signifikanten Vorteil der erfindungsgemäß erzielten, belastungsabhängigen Zusatzkinematik gegenüber der herkömmlichen belastungsunabhängigen Grundkinematik deutlich. Während bei Inanspruchnahme der Grundkinematik bei einem Beugewinkel α1 = 20° der Momentandrehpunkt P1 dicht oberhalb des Gelenkes und vor seiner streckseitigen Begrenzung liegt, ergibt sich bei einem belastungsabhängigen Beugewinkel β1 = 20° ein Momentandrehpunkt P′1, der weit entfernt oberhalb des Gelenkes und hinter seiner beugeseitigen Begrenzung liegt. Noch anschaulicher wird dies anhand der Figur 6, in die die Grundgeometrie der Schwenkverbindung gemäß Figur 5 maßstabsgetreu übernommen wurde. Das unter Einwirkung der zur Ferse gerichteten Belastung F in der Strecklage generierte Kniestreckmoment M₀ = F x α₀ erhöht sich im Falle einer Kniebeugung durch Nutzung der belastungsabhängigen Zusatzkinematik auf mehr als den doppelten Wert M′₁ = F x α′₁. Im Falle einer Kniebeugung durch bloße Nutzung der belastungsunabhängigen Grundkinematik einer herkömmlichen Konstruktion ergäbe sich hingegen ein extrem hohes, umgekehrt gerichtetes Beugemoment, das muskulär nicht mehr kompensierbar wäre und demzufolge im Praxisfall zwangsläufig zum Stolpern oder Sturz des Amputierten führen würde.

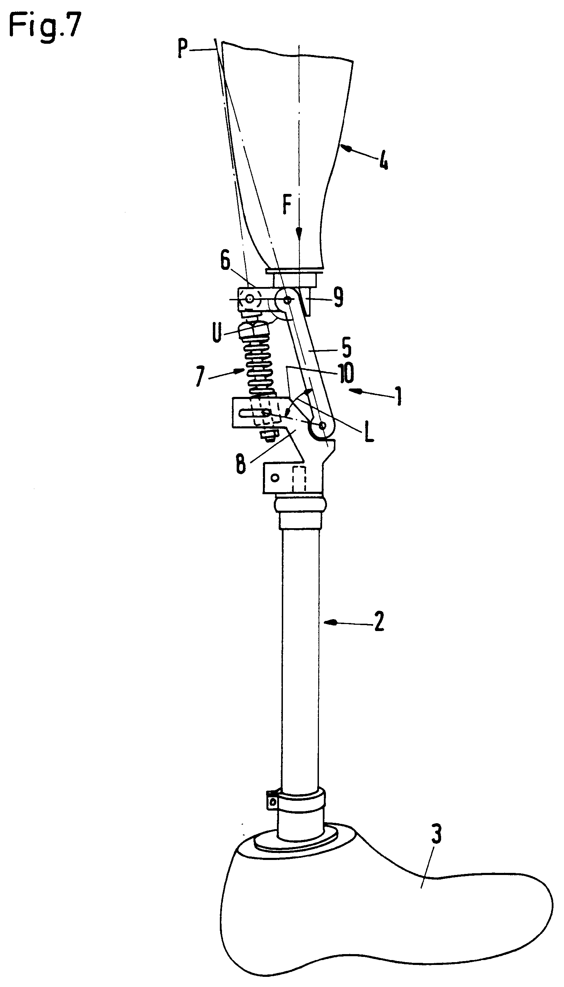

Die Figuren 7 bis 10 zeigen ein spezielles Ausführungsbeispiel für die Anwendung der erfindungsgemäßen Schwenkverbindung bei einem Prothesenkniegelenk.

Das Kniegelenk 1 verbindet einen Prothesenunterschenkel 2 mit einem Prothesenoberschenkel 4. Das Kniegelenk 1 wird im wesentlichen gebildet durch eine ebene, aus vier Gelenkgliedern 5,6,7,8 bestehende kinematische Gelenkkette. Das obere Gelenkglied 6 ist fest mit dem oberen Prothesenteil 4 und das untere Gelenkglied 8 fest mit dem unteren Prothesenteil 2 verbunden. Das in der Zeichnung links dargestellte Gelenkglied 7 ist als unter Belastung verkürzbares beugeseitiges Koppelglied ausgebildet. Der Momentandrehpunkt P des unteren Prothesenteils 2 gegenüber dem oberen Prothesenteil 4 ergibt sich aus dem Schnittpunkt der strichpunktiert eingezeichneten Verlängerungen der beiden Koppelglieder 5,7. Ein erster Schwenkanschlag 9 begrenzt die Verschwenkung des Koppelgliedes 5 gegenüber dem oberen Gelenkglied 6 und definiert dadurch die äußerste Streckstellung des Kniegelenkes 1, das außerdem noch einen zweiten Schwenkanschlag 10 aufweist, der den kleinsten Winkel zwischen den Gelenkgliedern 5,8 definiert und dadurch die Verkürzung des Koppelgliedes 7 begrenzt. In Figur 7 ist der zwischen dem streckseitigen Koppelglied 5 und dem oberen Gelenkglied 6 definierten Winkel mit U und der zwischen dem streckseitigen Koppelglied 5 und dem unteren Gelenkglied 8 eingeschlossene Winkel mit L bezeichnet.

Die in den Figuren 7 bis 10 dargestellte Prothese ist im einzelnen wie folgt aufgebaut:

Der Prothesenunterschenkel 2 setzt sich zusammen aus einem Modularteil, an dessen unterem Ende ein Prothesenfuß 3 befestigt ist. Das obere Prothesenteil 4 kann für die Verbindung mit einem Oberschenkelstumpf bzw. einem Knie-Exartikulationsstumpf gestaltet sein.

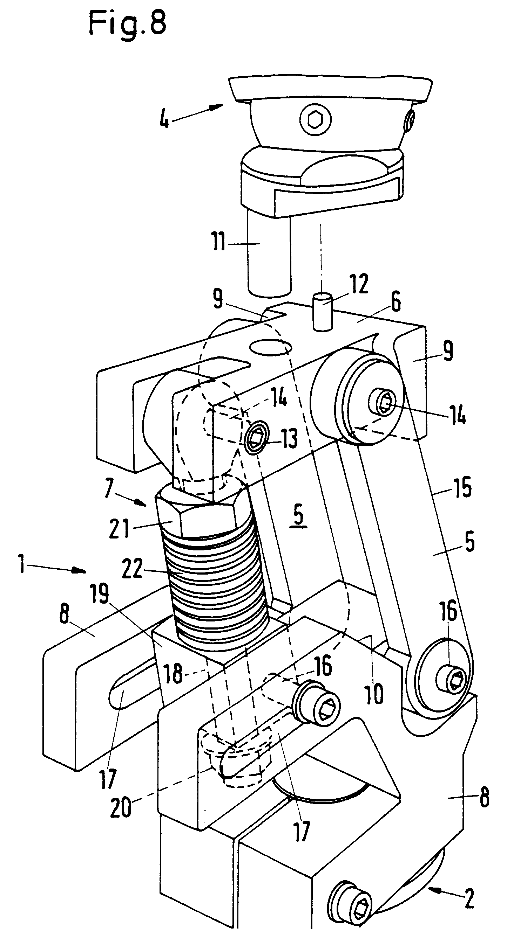

Das obere Gelenkglied 6 ist über einen Bolzen 11 und einen Stift 12 drehfest an einem Kupplungsteil des oberen Prothesenteils 4 befestigt (siehe insbesondere Figur 8). Die Koppelglieder 5,7 sind mit ihrem jeweils oberen Ende über in Bohrungen 13 des oberen Gelenkgliedes 6 angeordnete Schwenkeinrichtungen 14 am oberen Gelenkglied 6 verschwenkbar angelenkt. Der erste Schwenkanschlag 9 ist fest am oberen Gelenkglied 6 angeordnet, wirkt mit der Vorderfläche 15 des streckseitigen Koppelgliedes 5 zusammen (Fig. 8) und begrenzt dessen Schwenkwinkel U (siehe Figur 7). Dieser erste Schwenkanschlag 9 kann - obwohl in der Zeichnung nicht dargestellt - verstellbar ausgebildet sein, so daß sich die Grund-Geometrie der viergliedrigen Gelenkkette sowie die Lage ihres Momentandrehpunktes in der Stecklage verändern lassen, um die Grundjustierung der Prothese den individuellen Bedürfnissen des Amputierten in Bezug auf Sicherheit und Dynamik anpassen zu können

Die beiden Koppelglieder 5,7 sind mit ihrem unteren Ende über je eine Schwenkeinrichtung 16 am unteren Gelenkglied 8 angelenkt, das klemmend am oberen Ende des Modularteils 2 befestigt ist. Die das untere Ende des beugeseitigen Koppelgliedes 7 anlenkende Schwenkeinrichtung 16 ist durch Längsschlitze 17 im unteren Gelenkglied 8 geführt und kann in der gewünschten Position innerhalb der Längsschlitze 17 fixiert werden. Dadurch läßt sich die Lage des beugeseitigen Koppelgliedes 7 gegenüber dem streckseitigen Koppelglied 5 durch manuelle Längenveränderung des unteren Gelenkgliedes 8 variieren, um insbesondere die Höhenposition des Momentandrehpunktes P und damit das Stabilitätsgesamtverhalten des Kniegelenkes 1 zu optimieren.

Das beugeseitige Koppelglied 7 umfaßt einen Bolzen 18, der mit seinem unteren Ende längsverschiebbar geführt ist in einem auf der Schwenkeinrichtung 16 verschwenkbar gelagerten Stützkörper 19. Auf das untere Ende des Bolzens 18 ist eine Mutter 20 geschraubt, die im unbelasteten Zustand der Schwenkbewegung die effektive Grundlänge des Koppelgliedes 7 bestimmt, die verstellbar ausgebildet sein kann - z.B. durch Verwendung einer weiteren (Konter-) Mutter - , um eine zusätzliche Justiermöglichkeit zu erhalten.

Auch auf das obere Ende des Bolzens 18 ist eine Mutter 21 geschraubt, die gegenüber dem den Bolzen 18 an der oberen Schwenkeinrichtung 14 anlenkenden Bolzenauge verstellbar sein kann und das eine Widerlager für eine Druckfeder 22 bildet, die sich mit ihrem unteren Ende am Stützkörper 19 abstützt. Durch Verdrehen der oberen Mutter 21 läßt sich die Vorspannung der Druckfeder 22 justieren.

Wird das Kniegelenk 1 z.B. durch das Körpergewicht des Prothesenträgers belastet, erfolgt ein Zusammendrücken der Druckfeder 22 unter gleichzeitiger Verschiebung des Bolzens 18 gegenüber dem Stützkörper 19. Dadurch wird der Abstand zwischen den beiden Schwenkeinrichtungen 14,16 und somit die für die Kinematik des Gelenkes wichtige effektive Länge des beugeseitigen Koppelgliedes 7 verringert. Als Folge hiervon ergibt sich eine Veränderung der Lage des Momentandrehpunktes.

Das beugeseitige Koppelglied 7 könnte aber auch z.B. als Blattfeder ausgebildet sein, die sich unter Belastung stärker durchbiegt, wodurch ebenfalls der Abstand der beiden Schwenkpunkte 14, 16 voneinander reduziert würde.

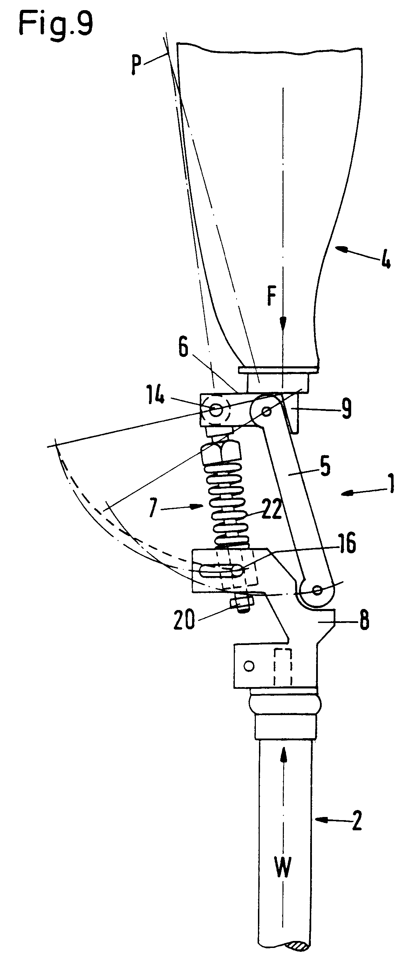

Befindet sich die Prothese in gestrecktem Zustand (Figur 9) und wird in dieser Lage z.B. vom Gewicht F des Prothesenträgers belastet, so ist es von großer Bedeutung, daß der Momentandrehpunkt P hinter der Belastungslinie liegt, da nur so vermieden werden kann, daß die mehrgliedrige Gelenkkette unter Einfluß der Belastung eine Verschwenkung entsprechend ihrer Grundkinematik ausführt. Da im gestreckten Zustand die Lage der Gelenkglieder 5,6 zueinander durch den ersten Schwenkanschlag 9 vorgegeben ist, erfolgt aufgrund der Verkürzung der effektiven Länge des Gelenkgliedes 7 eine Verschiebung des Momentandrehpunktes P auf einer Linie, die sich aus einer Verlängerung des vorderen Gelenkgliedes 5 bzw. seiner Verbindungslinie zwischen seinen beiden Gelenkpunkten 14,16 ergibt. Zur Erzielung einer möglichst großen Stabilität ist es wichtig, daß diese Linie immer hinter der Wirkungslinie der Belastung F liegt.

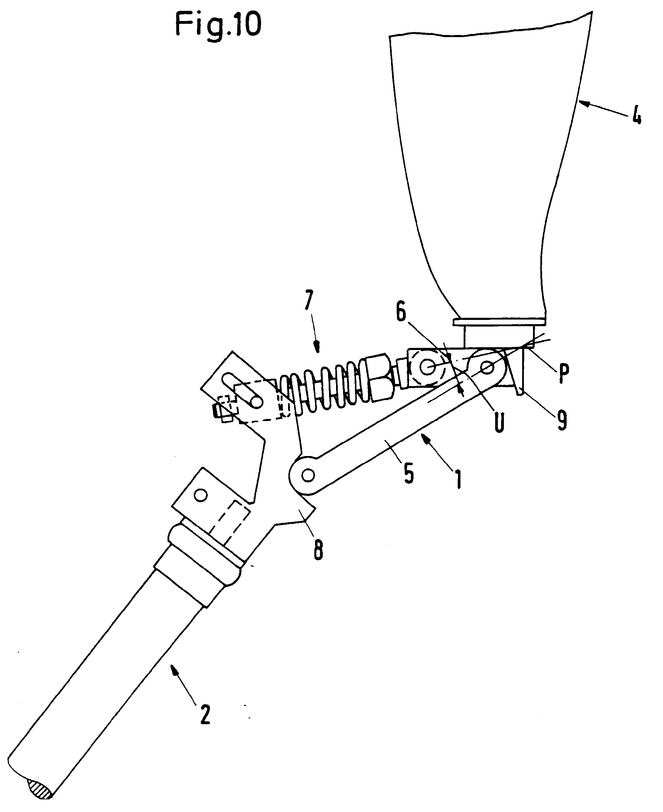

Für den Tragekomfort einer Beinprothese mit polyzentrischem Kniegelenk ist es von großer Bedeutung, daß der Momentandrehpunkt P mit zunehmendem Kniebeugewinkel - wie er sich insbesondere während der Schwungphase oder in der Sitzposition einstellt - in eine knienahe Position rückt, wie es Figur 10 erkennen läßt.

## Patentansprüche

1. Schwenkverbindung zwischen zwei Teilen eines orthopädietechnischen Hilfsmittels, z.B. einer Prothese oder Orthese,mit einer einrichtbaren, inhärenten Gelenk-Grundstabilität, bestehend aus einer ebenen kinematischen Gelenkkette mit zumindest vier Gelenkgliedern (5,6,7,8) und einer sich aus translatorischen und rotatorischen Komponenten zusammensetzenden polyzentrischen Verschwenkcharakteristik, wobei die Verschwenkung zumindest eines Gelenkgliedes in zumindest einer Schwenkrichtung durch einen ersten Schwenkanschlag (9) begrenzt ist, **dadurch gekennzeichnet,** daß unter Einwirkung einer äußeren Kraft (F) zumindest ein Gelenkglied (79 derart längenveränderlich ausgebildet ist, daß sich die Verschwenkcharakteristik in Abhängigkeit der äußeren Kraft (F) ändert.

2. Schwenkverbindung nach Anspruch 1, **dadurch gekennzeichnet,** daß ein zwei Gelenkglieder (7,8) miteinander verbindender Gelenkpunkt (C) unter Belastung in Richtung des einen und/oder des anderen Gelenkgliedes (7,8) verschiebbar ist.

3. Schwenkverbindung nach Anspruch 1 oder 2, gekennzeichnet durch eine der genannten Längenänderung entgegenwirkende Widerstandseinrichtung.

4. Schwenkverbindung nach Anspruch 1, 2 oder 3, dadurch gekennzeichnet, daß die Längenveränderung des zumindest einen Gelenkgliedes (7) federelastisch ausgebildet ist.

5. Schwenkverbindung nach einem der vorhergehenden Ansprüche, dadurch gekennzeichnet, daß der erste Schwenkanschlag (9) justierbar ist.

6. Schwenkverbindung nach einem der vorhergehenden Ansprüche, dadurch gekennzeichnet, daß der erste Schwenkanschlag (9) zwischen zwei starr ausgebildeten Gelenkgliedern (5,6) angeordnet ist.

7. Schwenkverbindung nach einem der vorhergehenden Ansprüche, dadurch gekennzeichnet, daß das Maximum der Verkürzung des Gelenkgliedes bzw. der Verschiebung des Gelenkpunktes (C) durch einen Längenanschlag (30) begrenzt ist.

8. Schwenkverbindung nach einem der Ansprüche 1 bis 6, dadurch gekennzeichnet, daß das Maximum der Verkürzung des Gelenkgliedes bzw. der Verschiebung des Gelenkpunktes (C) durch einen zweiten Schwenkanschlag (10) begrenzt ist. (Figuren 7 und 8)

9. Schwenkverbindung nach Anspruch 7 oder 8, dadurch gekennzeichnet, daß der Längen- und/oder zweite Schwenkanschlag (30,10) justierbar ist.

10. Schwenkverbindung nach einem der vorhergehenden Ansprüche, wobei das erste Teil des orthopädie-technischen Hilfsmittels einen Prothesenoberschenkel (4), das zweite Teil einen Prothesenunterschenkel (2) und eine viergliedrige Gelenkkette das Kniegelenk (1) bilden, dessen die beiden mit dem Prothesenober- bzw. -unterschenkel (4,2) fest verbundenen Gelenkglieder (6,8) über ein vorderes, für die Vorgabe der Strecklage genutztes Gelenkglied (streckseitiges Koppelglied 5) sowie über ein hinteres Gelenkglied (beugeseitiges Koppelglied 7) über Gelenkpunkte (A,B,C,D) miteinander verbunden sind, **dadurch gekennzeichnet,** daß das beugeseitige Koppelglied (7) das unter Belastung federelastisch längenveränderliche Gelenkglied ist.

11. Schwenkverbindung nach Anspruch 10, dadurch gekennzeichnet, daß der erste Schwenkanschlag (9) das streckseitige Koppelglied (5) beaufschlagt und das Maximum des zwischen dem oberen Gelenkglied (6) und dem streckseitigen Koppelglied (5) eingeschlossenen Winkels (U) begrenzt.

12. Schwenkverbindung nach Anspruch 10 oder 11, dadurch gekennzeichnet, daß sich das beugeseitige Koppelglied (7) über eine Druckfeder (22) an dem Gelenkpunkt (C) mit dem unteren Gelenkglied (8) abstützt.

13. Schwenkverbindung nach Anspruch 12, dadurch gekennzeichnet, daß die Vorspannung der Druckfeder (22) justierbar ist.

14. Schwenkverbindung nach einem der Ansprüche 10 bis 13, dadurch gekennzeichnet, daß der das beugeseitige Koppelglied (7) mit dem unteren Gelenkglied (8) verbindende Gelenkpunkt (C) innerhalb des unteren Gelenkgliedes (8) längsverschiebbar angeordnet und in der gewünschten Position fixierbar ist.

15. Schwenkverbindung nach Anspruch 14, dadurch gekennzeichnet, daß die genannte Längsverschiebbarkeit auf einem Kreisbogenabschnitt mit dem oberen Gelenkpunkt (D) als Mittelpunkt verläuft.

16. Schwenkverbindung nach einem der vorhergehenden Ansprüche, dadurch gekennzeichnet, daß bei gestrecktem Gelenk die Längenveränderung des Gelenkgliedes (7) bzw. die Längsverschiebung des Gelenkpunktes (C) angenähert in Richtung des bzw. parallel zum Belastungsvektor (F) erfolgen.

17. Schwenkverbindung nach einem der vorhergehenden Ansprüche, dadurch gekennzeichnet, daß die im unbelasteten Zustand der Schwenkverbindung wirksame Grundlänge des beugeseitigen Koppelgliedes (7) verstellbar ist.

## Claims

1. Swivel connection between two components of an orthopaedic aid, e.g. a prosthesis or orthotic device, having an adjustable, intrinsic basic joint stability and comprising a planar kinematic link chain having at least four link members (5, 6, 7, 8) and polycentric swivel characteristics composed of translatory and rotatory components, swivelling in at least one link member being limited in at least one swivel direction by a first swivel stop (9), **characterized in that,** under the action of an external force (F), at least one link member (7) is constructed to be variable in length such that the swivel characteristics alter as a function of the external force (F).

2. Swivel connection according to Claim 1, characterized in that a link point (C) connecting two link members (7, 8) to one another can be displaced under load in the direction of the one and/or the other link member (7, 8).

3. Swivel connection according to Claim 1 or 2, characterized by a resistance device which counteracts the change in length mentioned.

4. Swivel connection according to Claim 1, 2 or 3, characterized in that the change in length of at least the one link member (7) is constructed to be spring-elastic.

5. Swivel connection according to one of the preceding Claims, characterized in that the first swivel stop (9) is adjustable.

6. Swivel connection according to one of the preceding Claims, characterized in that the first swivel stop (9) is located between two rigidly constructed link members (5, 6).

7. Swivel connection according to one of the preceding claims, characterized in that the maximum shortening of the link member or displacement of the link point (C) is limited by a length stop (30).

8. Swivel connection according to one of Claims 1 to 6, characterized in that the maximum shortening of the link member or displacement of the link point (C) is limited by a second swivel stop (10). (Figures 7 and 8)

9. Swivel connection according to Claim 7 or 8, characterized in that the length and/or second swivel stop (30, 10) is adjustable.

10. Swivel connection according to one of the preceding Claims, in which the first component of the orthopaedic aid forms a prosthetic thigh (4), the second component forms a prosthetic lower leg (2) and a four-membered link chain forms the knee joint (1), whereof the two link members (6, 8), which are rigidly connected to the prosthetic thigh and lower leg (4, 2) respectively, are joined to one another via link points (A, B, C, D) via a front link member used for determining the extended position (coupling member 5 on the extensor side) and via a rear link member (coupling member 7 on the flexor side), **characterized in that** the coupling member (7) on the flexor side is the link member which can undergo a spring-elastic change in length under load.

11. Swivel connection according to Claim 10, characterized in that the first swivel stop (9) acts upon the coupling member (5) on the extensor side and limits the maximum of the angle (U) enclosed between the upper link member (6) and the coupling member (5) on the extensor side.

12. Swivel connection according to Claim 10 or 11, characterized in that the coupling member (7) on the flexor side is supported at the link point (C) with the lower link member (8) via a pressure spring (22).

13. Swivel connection according to Claim 12, characterized in that the initial tension of the pressure spring (22) is adjustable.

14. Swivel connection according to one of Claims 10 to 13, characterized in that the link point (C) which connects the coupling member (7) on the flexor side to the lower link member (8) is located such that it can be displaced longitudinally within the lower link member (8) and can be fixed in the desired position.

15. Swivel connection according to Claim 14, characterized in that the said longitudinal displaceability takes effect over an arc of a circle with the upper link point (D) as the centre point.

16. Swivel connection according to one of the preceding Claims, characterized in that with the joint extended, the longitudinal change in the link member (7) or the longitudinal displacement in the link point (C) occurs approximately in the direction of or parallel to the load vector (F).

17. Swivel connection according to one of the preceding Claims, characterized in that the basic length of the coupling member (7) on the flexor side effective in the non-loaded state of the swivel connection is adjustable.

## Revendications

1. Joint articulé entre deux parties d'un accessoire orthopédique, par exemple une prothèse ou une orthèse, présentant une stabilité intrinsèque et réglable de l'articulation, constitué d'une chaîne d'articulation cinématique plane comprenant au moins quatre éléments articulés (5, 6, 7, 8) et une caractéristique de pivotement polycentrique formée de composants translationnels et rotationnels, le pivotement d'au moins un des éléments de l'articulation étant limité dans au moins un sens de pivotement par une première butée d'articulation (9), caractérisé en ce qu'au moins un élément (7) de l'articulation est configuré de manière à être modifiable en longueur sous l'action d'une force extérieure (F), de sorte que sa caractéristique de pivotement se modifie en fonction de la force extérieure (F).

2. Joint articulé selon la revendication 1, caractérisé en ce qu'un point articulé (C) reliant mutuellement deux éléments (7, 8) de l'articulation peut sous contrainte se déplacer en direction de l'un et/ou de l'autre élément (7, 8) de l'articulation.

3. Joint articulé selon la revendication 1 ou 2, caractérisé par un dispositif résistant s'opposant à ladite modification de longueur.

4. Joint articulé selon la revendication 1, 2 ou 3, caractérisé en ce que la modification de longueur de l'élément (7) de l'articulation s'effectue sous le rappel élastique d'un ressort.

5. Joint articulé selon l'une au moins des revendications précédentes, caractérisé en ce que la première butée (9) de l'articulation est ajustable.

6. Joint articulé selon l'une au moins des revendications précédentes, caractérisé en ce que la première butée (9) de l'articulation est disposée entre deux éléments (5, 6), rigides, de l'articulation.

7. Joint articulé selon l'une au moins des revendications précédents, caractérisé en ce que le raccourcissement maximum de l'élément de l'articulation et le déplacement maximum du point d'articulation (C) sont limités par une butée de longueur (30).

8. Joint articulé selon l'une des revendications 1 à 8, caractérisé en ce que le raccourcissement maximum de l'élément de l'articulation et le déplacement maximum du point d'articulation (C) sont limités par une seconde butée de pivotement (10). (Figures 7 et 8).

9. Joint articulé selon la revendication 7 ou 8, caractérisé en ce que la première butée de longueur (30) et/ou la seconde butée de pivotement (10) sont ajustables.

10. Joint articulé selon l'une au moins des revendications précédentes, dont la première partie de l'accessoire orthopédique forme une cuisse (4) de la prothèse, dont la seconde partie forme une jambe (2) de la prothèse, une chaîne d'articulation à quatre éléments formant l'articulation du genou (1) dont les deux éléments (6, 8) fixés respectivement sur la cuisse (4) et sur la jambe (2) de la prothèse sont reliés l'un à l'autre en des points (A, B, C, D) d'articulation par l'intermédiaire d'un élément antérieur (élément d'accouplement (5) situé côté extension) utilisé pour délimiter l'ampleur de la position d'extension ainsi que par l'intermédiaire d'un élément postérieur (élément d'accouplement (7) situé côté flexion), caractérisé en ce que 1' élément d'accouplement (7) situé côté flexion constitue l'élément dont la longueur se modifie sous l'action d' une contrainte et sous le rappel élastique d'un ressort.

11. Joint articulé selon la revendication 10, caractérisé en ce que la première butée de pivotement (9) agit sur l'élément d'accouplement (5) situé côté extension et limite l'amplitude maximale de l'angle (U) formé entre l'élément supérieur (6) de l'articulation et l'élément d'accouplement (5) situé côté extension.

12. Joint articulé selon la revendication 10 ou 11, caractérisé en ce que l'élément d'accouplement (7) situé côté flexion s'appuie sur l'élément inférieur (8) de l'articulation, au point d'articulation et par l'intermédiaire d'un ressort de compression (22).

13. Joint articulé selon la revendication 12, caractérisé en ce que la précontrainte du ressort (22) est ajustable.

14. Joint articulé selon l'une des revendications 10 à 13, caractérisé en ce que le point (C) d'articulation reliant l'élément d'accouplement (7) situé côté flexion à l'élément inférieur (8) de l'articulation est disposé à déplacement longitudinal à l'intérieur de l'élément inférieur (8) de l'articulation et peut être immobilisé en position voulue.

15. Joint articulé selon la revendication 14, caractérisé en ce que ledit déplacement longitudinal s'effectue suivant un arc de cercle dont le point supérieur (D) de l'articulation constitué le centre.

16. Joint articulé selon l'une des revendications précédentes, caractérisé en ce que, lorsque l'articulation est en extension, la modification de longueur de l'élément (7) de l'articulation et le déplacement longitudinal du point (7) de l'articulation s'effectuent sensiblement parallèlement à la direction du vecteur de la contrainte (F).

17. Joint articulé selon l'une des revendications précédentes, caractérisé en ce que, lorsque le joint articulé n'est pas en charge, la longueur efficace de base de l'élément d'accouplement (7) est réglable.
